# EUROPEAN PATENT APPLICATION

(11) **EP 4 549 911 A1**
(43) Date of publication of application: **07.05.2025**
(21) Application number: 23829810.3
(22) Date of filing: 25.05.2023
(51) Int. Cl.: G01N 21/01, G01N 21/64, G01N 21/76, G01N 21/84

(54) **SINGLE-MOLECULE DETECTION METHOD BASED ON GRATING SCANNING RECOGNITION**

(30) Priority: 29.06.2022 CN 202210749511; 29.06.2022 CN 202210749471
(71) Applicant: Hunan Targeting Detection Technology Co., Ltd., Changsha, Hunan 410013 (CN)
(72) Inventor: XIE, Chengkun, Changsha, Hunan 410013 (CN); LI, Chaohui, Changsha, Hunan 410013 (CN); HE, Feng, Changsha, Hunan 410013 (CN); HUANG, Xin, Changsha, Hunan 410013 (CN); TAO, Lian, Changsha, Hunan 410013 (CN)
(74) Representative: Zhu, Junyi
(86) International application number: PCT/CN2023/096311
(87) International publication number: WO 2024/001625

(57) **Abstract**

A single molecule detection method based on grating scanning recognition, in which a large number of capture beads are scattered on a bead retaining plate (or a detection chip). At least part of the capture beads are specifically bound with an analyte, and each analyte is bound with a luminescent substance. Grating ports of a grating detection device are used to scan the capture beads column by column from the capture beads arranged at one end. The number of the capture beads bound with a marker is synchronously counted. The capture beads on the whole bead retaining plate are scanned through transverse relative movement between the grating ports and the capture beads. The total number of analytes can be obtained after scanning is completed. **In** the present invention, the number of detected substances is obtained in real time by counting the capture beads while scanning, in combination with the number of capture beads with a marker, which greatly improves the detection speed.

## Description

### Technical Field

The present invention relates to a detection method, particularly to a single molecule detection method based on grating scanning recognition, and belongs to the technical field of single molecule detection.

### Background

Single molecule detection (SMD) is an ultra-sensitive detection technology developed rapidly in recent years, which means to determine and analyze a target object at single molecule level. SMD is a brand new detection method, and also opens up a brand new detection field. Such a detection method can detect an analyte with a very low concentration, and amount of reagents required and space occupied by detection are very small. One example is "CN202080000774.8 - a single molecule quantitative detection method and detection system" disclosed by Suzhou ASTRABIO Co., Ltd., which is to introduce in situ signal-enhanced nanoparticles with an optical effect during immune recognition to mark molecules to be detected, enhance signals generated by an original luminescent material to improve differentiation degree, and then use optical imaging equipment to obtain the number of molecules to be detected having light spots. Another example is "CN201180019462.2 - ultrasensitive detection of molecules or particles using microbeads or other captured substances" disclosed by American Quanterix, which is to divide captured substances into a certain space, then form an image of the captured substances, and count the number of luminescent analytes in the image to determine the concentration. The methods can greatly improve sensitivity of detection. However, the above methods are to count the number of luminescent analytes in a picture after imaging, which has a high requirement for imaging equipment and a low detection speed.

### Summary

In view of the problems that an existing single molecule detection method adopts an imaging method to calculate the concentration of analytes, which has a high requirement for imaging equipment and a low detection speed, the present invention provides a single molecule detection method based on grating scanning recognition, which obtains the number of captured analytes in real time by means of grating scanning, and has the advantages of simple equipment and high detection speed.

The technical means adopted by the present invention to solve the above problems is: a single molecule detection method based on grating scanning recognition, in which a large number of capture beads are scattered on a bead retaining plate (or a detection chip), wherein at least part of the capture beads are specifically bound with an analyte, and each analyte is bound with a luminescent substance, grating ports of a grating detection device are used to scan the capture beads column by column from the capture beads arranged at one end, the number of the capture beads bound with a marker is synchronously counted, the capture beads on the whole bead retaining plate are scanned through transverse relative movement between the grating ports and the capture beads, and the total number of analytes can be obtained after scanning is completed.

Further, one grating detection device comprises a grating head, an optical sensor and a signal processor, wherein the grating head comprises a plurality of independent grating ports arranged in columns, and the optical sensor comprises a plurality of independent sensing elements; when scanning, one grating port is aligned with or not aligned with one capture bead, and one sensing element is corresponding to one grating port and can sense incident light at the grating port; when the sensing element senses the incident light, a signal is fed back to the signal processor, and the number of analytes is counted by the signal processor according to a feedback result.

Further, the sensing elements include photosensitive tubes, which output pulse signals to the signal processor after sensing the incident light, and the number of analytes is counted by the signal processor according to the number of photosensitive tubes that output pulse signals.

Further, the grating ports of one grating detection device are arranged in one column, the number of the grating ports in the whole column is greater than or equal to the number of rows of the capture beads arranged on the bead retaining plate, and the distance between centers of two adjacent grating ports is equal to the distance between centers of two adjacent rows of capture beads in the same column.

Further, the grating ports of one grating detection device are arranged in a stagger layout in two columns, the total number of the grating ports in the two columns is greater than or equal to the number of rows of the capture beads on the bead retaining plate, the distance between centers of two adjacent grating ports in each column is equal to the distance between centers of two rows of capture beads separated by one row in the same column, and two columns of grating ports are matched with one column of capture beads for scanning.

The stagger layout refers to that the positions of two adjacent grating ports form a V-shape; when one column of grating ports is aligned with several rows of capture beads on the bead retaining plate, the other column of grating ports is aligned with the adjacent rows of capture beads; that is, two adjacent rows of capture beads arranged in the same column will not be aligned with one column of grating ports at the same time, but will be aligned with the two columns of grating ports successively in the transverse movement between the capture beads and the grating ports along the rows.

Further, the distance between centers of two adjacent capture beads is greater than or equal to twice the outside diameter of the capture beads.

Further, the distance between centers of two adjacent capture beads is greater than or equal to three times the outside diameter of the capture beads.

Further, the distance between the grating ports and the surface of the capture beads is less than or equal to twice the outside diameter of the capture beads.

Further, the distance between the grating ports and the surface of the capture beads is equal to 1.5 times, 1 time or 0.5 times the outside diameter of the capture beads. **In** theory, the smaller the distance between the grating ports and the surface of the capture beads without contact with the substance on the surface of the capture beads, the better, so that the light emitted by the marker can be emitted into the grating ports as much as possible, inaccurate detection results caused by the loss of light when the distance is too large are avoided.

Further, the inside diameter of a single grating port is between 0.5 times the outside diameter of the capture beads and 1.5 times the outside diameter of the capture beads.

Further, the inside diameter of a single grating port is equal to the outside diameter of the capture beads. Preferably, the area of a light transmission region at a single grating port is close to the projected area of a luminescent region of the capture beads at the grating ports; at the same time, the distance between the grating ports and the surface of the capture beads is controlled in coordination, which ensures that the light emitted by the luminescent substance on the surface of the capture beads can be emitted into the grating ports in maximum quantity, and avoids that the light emitted by the luminescent substance on the surface of other capture beads at adjacent positions are emitted into the grating ports and affect the detection results.

Preferably, the capture beads are arranged on the bead retaining plate in an array to minimize the size of equipment, and the amount of light that can enter the grating ports is controlled, which can not only conduct the sensing elements to make the sensing elements send signals, but also prevent the light on the surface of adjacent capture beads from entering the grating port and make the sensing elements send wrong signals.

Further, the luminescent substance includes fluorescent or chemiluminescent reagents.

Further, when the luminescent substance is a fluorescent reagent, excitation light sources are arranged near the bead retaining plate to irradiate the capture beads.

Further, more than two analytes and more than two luminescent substances are used, with one analyte bound with one luminescent substance, and more than two grating detection devices are used, with one grating detection device counting one luminescent substance.

The present invention has the following beneficial effects:

1. In the present invention, the number of detected substances is obtained in real time by counting the capture beads while scanning, in combination with the number of capture beads with a marker, which greatly improves the detection speed.

2. In the present invention, light signals of the luminescent substances are converted into electrical signals during scanning, so that the method of counting the number of the detected substances in real time can be realized.

3. In the present invention, the grating ports are arranged in a stagger layout in two columns, which is equivalent to increasing the distance between adjacent grating ports, so that light interference between adjacent capture beads during synchronous detection is avoided, and the accuracy of detection results is improved.

### Description of Drawings

Fig. 1 is a schematic diagram of arranging magnetic beads on a microwell array plate in embodiment 1;
Fig. 2 is a schematic diagram of automatic scanning recognition in embodiment 1;
Fig. 3 is a schematic diagram of arrangement of grating ports of a grating head in embodiment 1;
Fig. 4 is a schematic diagram of arrangement of grating ports of a grating head in embodiment 2;
Fig. 5 is a schematic diagram of a corresponding relationship between a microwell array plate and a grating port when looking down at the microwell array plate and looking up at the grating port;
Fig. 6A-Fig. 6F are schematic diagrams of signals corresponding to detection of luminescent magnetic beads arranged according to different position relationships;
Fig. 7 is a schematic diagram of a position relationship between excitation light sources, capture beads and a grating detection device in embodiment 3;
Fig. 8 and Fig. 9 are schematic diagrams of arranging a grating detection device in two ways in embodiment 4;

In the figures: 10. microwell array plate, 11. microwell, 12. magnetic bead, 13. fluorescent marker, 20. grating detection device, 21. grating head, 211. grating port, 22. optical sensor, 23. signal processor, 24. excitation light source, 25. reflector, 26. bracket, and 27. rotating center.

### Detailed Description

The present invention is further described below in combination with the drawings.

### Embodiment 1

In the embodiment, a substance emitting fluorescence is used as a marker, magnetic beads are used as capture beads, a strip-shaped microwell array plate with a microwell array is used as a bead retaining plate, some microwells have a magnetic bead, respectively, some microwells have no magnetic bead, at most one analyte is specifically bound to the surface of each bead, and each analyte is bound with a fluorescent marker. The magnetic beads (capture beads) of adjacent rows or adjacent columns in the present invention refer to the magnetic beads (capture beads) at the positions where the microwells of adjacent rows or adjacent columns are located in the array, and if no magnetic bead is arranged in the microwells of adjacent rows or adjacent columns, the magnetic beads (capture beads) of adjacent rows or adjacent columns refer to the positions where the microwells of adjacent rows or adjacent columns are located in the array.

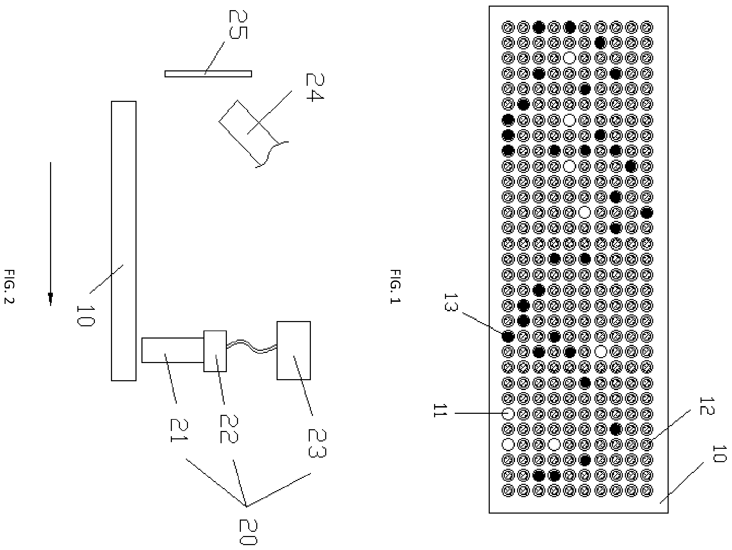

According to a single molecule detection method based on grating scanning recognition, as shown in Fig. 1, a determined number of magnetic beads 12 are arranged on a microwell array plate 10, some magnetic beads 12 are not bound with any analyte on the surface, and some magnetic beads 12 are bound with analytes, i.e., bounded with fluorescent markers 13; at most one magnetic bead 12 is arranged in each microwell 11, and no magnetic bead 12 is arranged in some microwells 11.

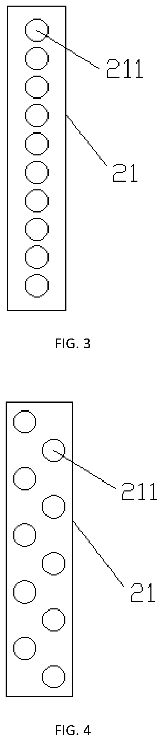

Then grating scanning recognition detection is carried out, as shown in Fig. 2, excitation light sources 24 are arranged above the microwell array plate 10, emitted excitation light is reflected by a reflector 25 and irradiated to the surface of the magnetic beads 12 to be scanned, and the excitation light is turned off when excitation is completed. A grating head 21 of a grating detection device 20 is arranged above (and of course can also be arranged below) the microwell array plate 10, and grating ports 211 of the grating head 21 are aligned with the magnetic beads 12, so that the fluorescence emitted by the fluorescent markers 13 after excitation can be emitted into the grating ports 211 to the maximum extent. As shown in Fig. 3, the number of the grating ports 211 shall be greater than or equal to the number of the microwells 11 in one column of the microwell array plate 10, and the distance between centers of two adjacent grating ports 211 shall be equal to the distance between centers of two adjacent microwells 11, so that the whole column of magnetic beads 12 of the microwell array plate 10 can be scanned and recognized at one time. At the same time, in order to correctly count the number of the grating ports 211 emitting fluorescence, an optical sensor 22 of the grating detection device 20 is arranged on another end of the grating head 21 opposite to the grating ports 211, and sensing elements (such as photosensitive tubes) of the optical sensor 22 are corresponding to the grating ports 211 one to one; when fluorescence is emitted into a grating port 211, a corresponding sensing element will receive the incident light and send a signal to a signal processor 23 of the grating detection device 20; if a photosensitive tube can generate a pulse signal as a signal after sensing the fluorescence, the signal processor 23 will receive the pulse signal for subsequent processing, and fluorescent magnetic beads 12 can be counted by counting the number of signals emitted by the optical sensor 22, thus to obtain the specific number of the analytes; of course, the method of converting optical signals into electrical signals and counting the electrical signals is easy to achieve by the prior art.

During scanning recognition, one column of magnetic beads 12 is detected each time, therefore, in order to complete the detection of all magnetic beads 12 in the microwell array, the microwell array plate 10 and the grating detection device 20 need to be moved relative to each other, so as to make the grating head 21 pass above (or below) each column of microwells 11 for detection. As shown in Fig. 2, the relative movement can be achieved by rightward transverse movement of the microwell array plate 10, and of course can also be achieved by leftward transverse movement the grating head 21.

In the detection method, the number of all magnetic beads 12 is determined when arranging the magnetic beads 12, and then the number of luminescent magnetic beads 12, i.e., the number of the analytes, is determined by scanning recognition, thus the proportion of the analytes can be known, and the concentration of the analytes in a test solution can be known by comparing with a standard concentration curve.

### Embodiment 2

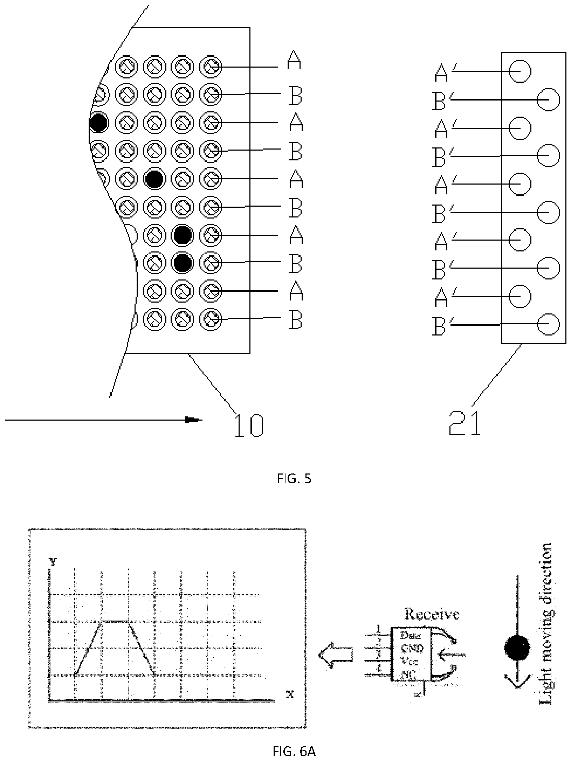

The principle of the embodiment is the same as that of embodiment 1, except that the arrangement of the grating ports 211 of the grating head 21 is different. As shown in Fig. 4, the grating ports 211 are arranged in a stagger layout in two columns, i.e., the positions of two adjacent grating ports 211 form a V-shape, thus to increase the distance between two adjacent grating ports 211; at this time, the sum of the numbers of grating ports 211 in two columns is greater than or equal to the number of the microwells 11 in one column. As shown in Fig. 5, during scanning recognition, after the grating head 21 is aligned with the microwell array plate 10 from above, the grating ports 211 marked as A' are located directly above the magnetic beads 12 marked as A and close to the magnetic beads 12, and the grating ports 211 marked as B' are located directly above the magnetic beads 12 marked as B and close to the magnetic beads 12. If the microwell array plate 10 is moved transversely from left to right during detection to achieve the relative movement between the microwell array plate 10 and the grating detection device 20, then at the beginning of scanning recognition, the grating head 21 is located above the microwells 11 in a rightmost column of the microwell array plate 10, the grating ports 211 marked as B' are aligned with the magnetic beads 12 marked B to be detected, the grating ports 211 marked as A' are not aligned with the microwells 11, and the magnetic beads 12 marked as A will not be detected at this time; when the detection of the magnetic beads 12 marked as B are completed by the grating ports 211 marked as B', the microwell array plate 10 is moved transversely from left to right to complete the relative movement between the microwell array plate 10 and the grating detection device 20; at this time, the grating ports 211 marked as B' are aligned with a next adjacent column of magnetic beads 12 in the same row as that marked as B, and the grating ports 211 marked as A' are aligned with the magnetic beads 12 marked as A, thus to synchronously complete the detection of the magnetic beads 12 at stagger positions in two adjacent columns. All magnetic beads 12 on the microwell array plate 10 are detected in this way. Therefore, due to the increase of the distance between adjacent grating ports 211, the light interference between adjacent magnetic beads 12 can be reduced, making the detection results more accurate.

### Detection result

Detection results are illustrated as follows according to the experimental results, and in order to simplify experimental operation and facilitate understanding, only one grating port 211 and one photosensitive tube are used for operation.

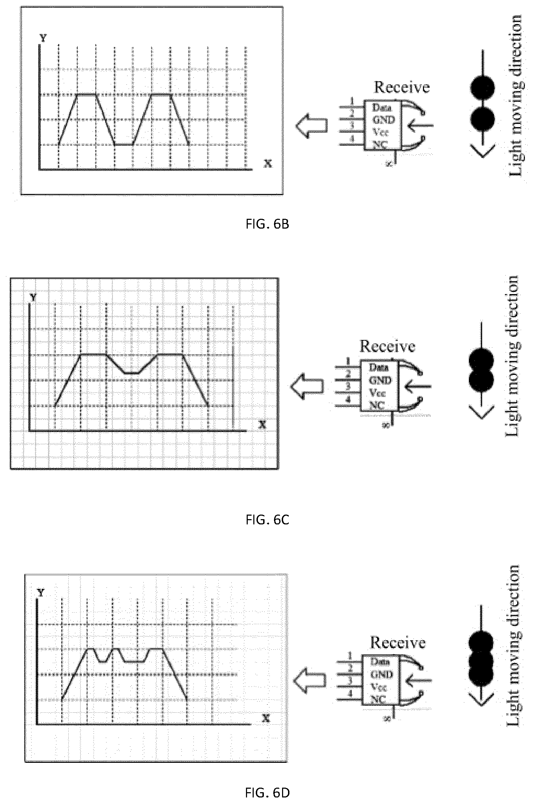

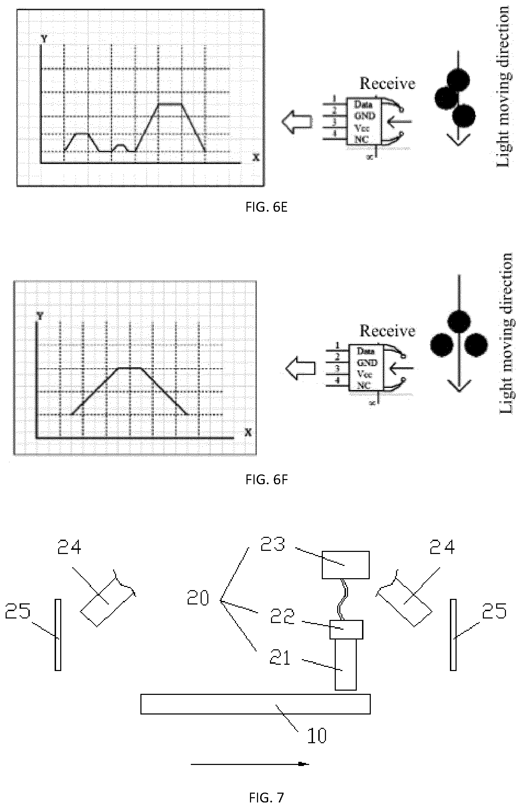

As shown in Fig. 6A, when a single luminescent capture bead passes below the grating port, a normal signal wave is sent out after processing by the signal processor. As shown in Fig. 6B, when two luminescent capture beads with a moderate distance pass below the grating port in sequence, two normal signal waves are sent out after processing by the signal processor, and the spacing between peak and trough is obvious and uniform. As shown in Fig. 6C, when two luminescent capture beads are too close to each other or even partially overlap at the edges, the peaks of two waves in the signal waves sent out after processing by the signal processor are too close, and the trough is not obvious. As shown in Fig. 6D, when three luminescent capture beads too close to each other pass below the grating port in sequence, the difference between peak and trough of signal waves sent out after processing by the signal processor is not obvious. As shown in Fig. 6E and 6F, when three luminescent capture beads are not in the same line, one of which is directly below the grating port, and the other two are near the grating port, the detection will be affected, and no normal signal wave can be formed.

Therefore, in order to ensure the accuracy of the detection results and achieve a compact detection system, capture beads with appropriate specifications are selected as far as possible, such as microbeads with a diameter of 2.7 um, and when arranging the capture beads, the distance between centers of two adjacent capture beads is controlled as far as possible, such as a distance of 2.5-3 times the diameter. At the same time, it is also necessary to minimize the size of the grating port, for example controlling the light transmission area within the range of the cross-sectional area of a capture bead, as long as the light transmitted is enough to conduct the sensing elements and enable the sensing elements to send out easily recognizable signals after signal processing. At the same time, the distance between the grating ports and the surface of the capture beads is as close as possible, and the light of adjacent luminescent capture beads is prevented from entering the grating ports as far as possible, that is, the light interference at adjacent positions is reduced.

Although the above embodiment adopts magnetic beads as capture beads, particles of other shapes with or without magnetism may also be adopted in actual use, the analytes can be either proteins or nucleic acid molecules, and the bead retaining plate can be in a structure with wells or slightly concave pits or even a plane. When the bead retaining plate has wells or pits, because the wells or pits are processed in advance, the wells or pits can be processed into a regular array form, the capture beads can be regularly placed into the wells or pits, and the grating ports arranged in columns are easy to match with the wells or pits; when the bead retaining plate is a plane, the capture beads can be arranged on the surface of the bead retaining plate by using some auxiliary structures, or the capture beads can be randomly scattered on the surface of the bead retaining plate. For the method of randomly scattering the capture beads on the plane, the distance between the capture beads shall be increased as much as possible in order to prevent the capture beads from being too close to each other or even agglomerating; therefore, when the same number of capture beads are scattered on the plane, the surface area of the bead retaining plate will be larger than that of the bead retaining plate with wells or pits, and the position relationship between the capture beads is largely disorder; however, in general, the capture beads can still be regarded as an array arranged into multiple rows and columns, except that many positions in the array have no capture bead and the capture beads in the same row or column are not strictly on a straight line; so the detection method of scanning column by column in embodiment 1 or embodiment 2 above can still be adopted.

### Embodiment 3

In the embodiment, as shown in Fig. 7, the bead retaining plate and the grating detection device 20 are arranged up and down, and two or more excitation light sources 24 and reflectors 25 are uniformly arranged on the periphery of the bead retaining plate; during detection, the excitation light sources 24 and the reflectors 25 remain stationary, and the bead retaining plate or the grating detection device 20 is moved in the space between the excitation light sources 24. Of course, the excitation light sources 24 can also be arranged in other ways, as long as it is ensured that the surface of each capture bead is irradiated and the marker on the surface of each capture bead can be excited to fluoresce.

### Embodiment 4

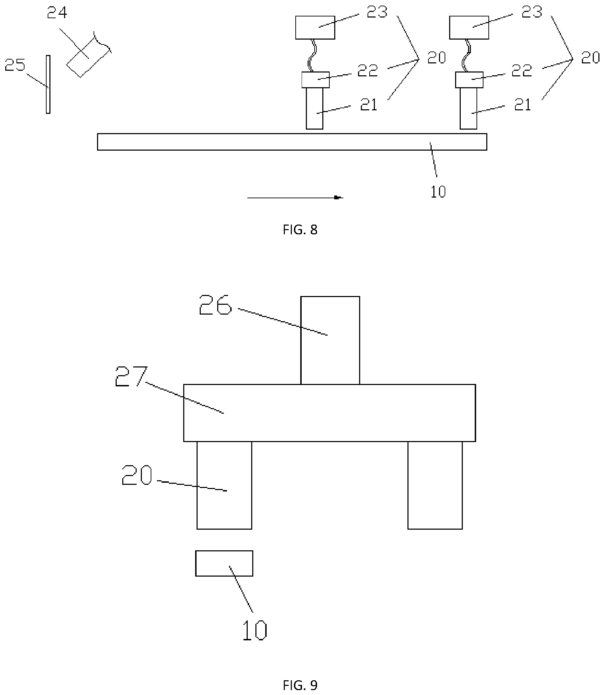

In the embodiment, more than two analytes can be detected on a single bead retaining plate; at this time, different analytes are bound with different luminescent substances, the excitation light sources 24 also need to be able to emit light with more than two wavelengths, and more than two grating detection devices 20 are arranged, with one grating detection device 20 detecting and counting one analyte. The more than two grating detection devices 20 can be arranged in a variety of ways:
(I) As shown in Fig. 8, each grating detection device 20 comprises a grating head 21, an optical sensor 22 and a signal processor 23, the more than two grating detection devices 20 are arranged in parallel rows, scanning is started from the capture beads at the same end of the bead retaining plate successively, and the scanning detection of all capture beads is completed through the relative movement between the bead retaining plate and the grating detection devices 20.
(II) As shown in Fig. 9, the more two grating detection devices 20 are arranged symmetrically on a supporting bracket 26 through a rotating center 27, and the rotating center 27 can be rotated around the bracket 26; when two grating detection devices 20 are used, the grating detection devices 20 are arranged at both ends of the rotating center 27; when three grating detection devices 20 are used, the included angle between two adjacent grating detection devices 20 is 120°; when more grating detection devices 20 are used, the grating detection devices 20 are also arranged symmetrically according to this law. During scanning detection, one analyte is scanned by one grating detection device 20 from one end of the bead retaining plate; when it is detected on the other end that the scanning detection of the analyte is completed, the rotating center 27 is rotated to move another grating detection device 20 to the end of the bead retaining plate, and the bead retaining plate or the bracket 26 is moved in a direction opposite to that in the previous detection process to complete the scanning detection of a second analyte. The process is repeated until the scanning detection of all analytes is completed.
(III) The implementation mode of more than two grating detection devices is: one set of grating head 21, optical sensor 22 and signal processor 23 is arranged, and filters at the grating ports of the grating head 21 are in replaceable structures; when scanning:
   Step 1: selecting required filters at the grating ports, starting scanning by one grating detection devices 20 from the capture beads at one end (an initial end) of the bead retaining plate, and scanning by the grating detection 20 to the capture beads at the other end (a final end) of the bead retaining plate through the relative movement between the bead retaining plate and the grating detection device 20 to complete the scanning detection of one marker;
   Step 2: replacing the filters at the grating ports, and scanning by the grating detection device 20 from the capture beads at the final end of the bead retaining plate to the initial end of the scanning in step 1 through the relative movement between the bead retaining plate and the grating detection device 20 in a direction opposite to that in the step 1 to complete the scanning detection of another marker;

The process is repeated until the scanning detection of all markers is completed.

The above embodiments are merely used for illustration of the present invention, and not intended to limit the present invention. Various changes or transformations can also be made by those skilled in the art without departing from the spirit and the scope of the present invention. Therefore, all equivalent technical solutions shall also belong to the protection scope of the present invention, and the protection scope of the present invention shall be defined by the claims.

## Claims

1. A single molecule detection method based on grating scanning recognition, **characterized in that**: a large number of capture beads are scattered on a bead retaining plate (or a detection chip), wherein at least part of the capture beads are specifically bound with an analyte, and each analyte is bound with a luminescent substance, grating ports of a grating detection device are used to scan the capture beads column by column from the capture beads arranged at one end, the number of the capture beads bound with a marker is synchronously counted, the capture beads on the whole bead retaining plate are scanned through transverse relative movement between the grating ports and the capture beads, and a total number of the analytes can be obtained after scanning is completed.

2. The single molecule detection method based on grating scanning recognition according to claim 1, **characterized in that**: the grating detection device comprises a grating head, an optical sensor and a signal processor, wherein the grating head comprises a plurality of independent grating ports arranged in columns, and the optical sensor comprises a plurality of independent sensing elements; when scanning, each one of the grating ports is aligned with or not aligned with one of the capture beads, and each one of the sensing elements is corresponding to one of the grating ports and can sense incident light at the grating port; when the sensing element senses the incident light, a signal is fed back to the signal processor, and a number of the analytes is counted by the signal processor according to a feedback result.

3. The single molecule detection method based on grating scanning recognition according to claim 2, **characterized in that**: the sensing elements comprise photosensitive tubes, which output pulse signals to the signal processor after sensing the incident light, and the number of the analytes is counted by the signal processor according to a number of photosensitive tubes that output pulse signals.

4. The single molecule detection method based on grating scanning recognition according to claim 2, **characterized in that**: the grating ports of the grating detection device are arranged in a column, a number of the grating ports in the whole column is greater than or equal to a number of rows of the capture beads arranged on the bead retaining plate, and a distance between centers of two adjacent grating ports is equal to a distance between centers of two adjacent rows of the capture beads in the same column.

5. The single molecule detection method based on grating scanning recognition according to claim 2, **characterized in that**: the grating ports of the grating detection device are arranged in a stagger layout in two columns, a total number of the grating ports in the two columns is greater than or equal to a number of rows of the capture beads on the bead retaining plate, a distance between centers of two adjacent grating ports in each column is equal to a distance between centers of two rows of the capture beads separated by one row in the same column, and the two columns of the grating ports are matched with one column of capture beads for scanning.

6. The single molecule detection method based on grating scanning recognition according to claim 1, **characterized in that**: a distance between centers of two adjacent capture beads is greater than or equal to twice an outside diameter of the capture beads.

7. The single molecule detection method based on grating scanning recognition according to claim 1, **characterized in that**: a distance between the grating ports and surfaces of the capture beads is less than or equal to twice an outside diameter of the capture beads.

8. The single molecule detection method based on grating scanning recognition according to claim 7, **characterized in that**: the distance between the grating ports and the surfaces of the capture beads is less than or equal to the outside diameter of the capture beads.

9. The single molecule detection method based on grating scanning recognition according to claim 1, **characterized in that**: an inside diameter of a single grating port is between 0.5 times an outside diameter of the capture beads and 1.5 times the outside diameter of the capture beads.

10. The single molecule detection method based on grating scanning recognition according to claim 1, **characterized in that**: more than two analytes and more than two luminescent substances are used, with each one of the analytes bound with one of the luminescent substances, and more than two grating detection devices are used, with each one of the grating detection devices counting one of the luminescent substances.
